(19) Europäisches Patentamt
European Patent Office
Office européen des brevets

(11) Publication number: **0 408 372 A1**

# EUROPEAN PATENT APPLICATION

(21) Application number: 90307658.6

(51) Int. Cl.5: **A61K 37/02**

(22) Date of filing: **12.07.90**

The applicant has subsequently filed a sequence listing and declared, that it includes no new matter.

(30) Priority: **13.07.89 US 379483**

(43) Date of publication of application:
**16.01.91 Bulletin 91/03**

(84) Designated Contracting States:
**AT BE CH DE DK ES FR GB GR IT LI LU NL SE**

(71) Applicant: **SMITHKLINE BEECHAM CORPORATION**
**One Franklin Plaza**
**Philadelphia Pennsylvania 19103(US)**

(72) Inventor: **Bugelski, Peter J.**
**439 East Wadsworth**
**Philadelphia, Pennsylvania 19119(US)**
Inventor: **Hart, Timothy K.**
**402-3B Strafford Avenue**
**Strafford, Pennsylvania 19087(US)**
Inventor: **Ellens, Harma**
**74 North Wakefield Road**
**Norristown, Pennsylvania 19403(US)**
Inventor: **Kirsh, Richard**
**581 Forest Road**
**Wayne, Pennsylvania 19087(US)** '

(74) Representative: **Giddings, Peter John, Dr. et al**
**SmithKline Beecham, Corporate Patents,**
**Mundells**
**Welwyn Garden City, Hertfordshire AL7 1EY(GB)**

(54) **Anti-viral therapy.**

(57) A gp 120 stripping agent for use in therapy, in particular in the treatment of HIV infection.

## ANTI-VIRAL THERAPY

The present invention relates to the field of anti-viral therapy and more particularly to the field of therapy for human immunodeficiency virus (HIV) infection.

HIV, the etiological agent of acquired immune deficiency syndrome (AIDS), shows a marked affinity for CD-4[+] lymphocytes. The human T-cell glycoprotein CD-4, sometimes previously referred to as "T4", is one of several non-polymorphic T lymphocyte surface receptor proteins that have been implicated in the mediation of efficient interactions of lymphocytes with other cells. Analysis of these surface proteins indicates that mature T lymphocytes segregate into two classes on the basis of their predominant expression of either the T4 or T8 surface glycoprotein. The CD-4 molecule is predominantly expressed on helper T lymphocytes whereas T8 is expressed on cytotoxic and suppressor T cells. The CD-4 lymphotropic character of the virus can be explained by its specific binding to the CD-4 receptor. Monoclonal antibodies directed against CD-4 block HIV infection of CD-4[+] cells in vitro . Human cells lines, nonlymphoid as well as lymphoid, which lack the CD-4 receptor cannot be infected by HIV, but these cells become susceptible to infection upon introduction and expression of the cloned CD-4 (T4) gene. AIDS results in the impairment, and ultimately the depletion, of CD-4[+] lymphocytes with consequent dysfunction of the cellular immune response.

Infection of cells by HIV is believed to occur following binding of the viral envelope glycoprotein, gp 120, to cellular CD4. sT4, a soluble recombinant form of CD4, binds gp120 and inhibits both viral infectivity and HIV-mediated syncytia formation.

gp 120 is synthesized as a 160 kd mw precursor which is cleaved proteolytically to gp 41 and gp120. Following cleavage, gp120 and gp 41 remain associated by non-covalent interaction. gp120 is shed from the surface of HIV-1 infected cells and virions and binds to surface CD4 on uninfected cells with high affinity which can result in their functional alteration. Cells coated with gp120 become targets for anti-gp120 antibodies and can be killed via antibody dependent cell-mediated cytotoxicity. gp120 has also been shown to directly suppress T cell function in in vitro assays.

A cDNA sequence of the human CD-4 (T-4) receptor has been described (Maddon, et al ., Cell 43 :93 (1985)). The complete CD-4 pre-protein sequence is 458 amino acids in length comprising the putative 23 amino acid secretory leader, 372 amino acid surface ($V_1$-$V_4$), 23 amino acid transmembrane and 40 amino acid cytoplasmic domains. The surface domains shows four regions of limited homology, 20-30%, to immunoglobulin variable (V) and joining (J) regions. Four of the five intron-exon boundaries in the surface domain occur near the junctions of these V-J regions. On the basis of partial protein sequence information for the mouse and sheep T4 proteins (Classon, et al ., Immunogen . 23:129 (1986), the 6 cysteines in the surface domain are paired sequentially to give three disulfide bonds. There are two possible sites for N-linked glycosylation based on amino acid sequence. The V-J homology, cysteine pairing and intron-exon structure suggest that CD-4 (T-4) shares some structural similarities with, the immunoglobulins.

Recently, a number of scientific investigators have reported their findings related to the interaction between soluble forms of CD-4 and the AIDS infection.

Deen, et al ., Nature 331 :82 (1988) report the isolation and expression of sCD-4 in several cellular environments. sCD-4 is a soluble derivative of CD-4 which lacks the transmembrane and cytoplasmic domains of CD-4.

Capon et al ., Nature 337 :525-531, (9 Feb. 1989) reported hybrid antibody-like molecules containing the gp120-binding domain of the receptor for HIV. These molecules block HIV-1 infection of T cells and monocytes, have a long plasma half-life and other antibody-like properties.

Watanabe et al ., Nature 337 :267-270, (19 Jan. 1989) reports effects of recombinant soluble CD4 in rhesus monkeys infected with simian immunodeficiency virus of macaques. Monkeys were given a 50 day course of treatment, receiving daily 2 mg intramuscular (i.m.) inoculations of recombinant CD4. Virus was readily isolated from peripheral blood lymphocytes and bone marrow cells of the animals before starting treatment with soluble CD4, but became difficult to isolate soon after treatment began.

There continues to be a need for methods of preventing and treating immune dysfunction associated with HIV infection.

The present invention provides a stripping agent which removes at least a portion of gp120 from the surface of HIV or virus infected cells to thereby decrease infectivity of the virus for use in therapy, in particular in the treatment of HIV infection. The stripping agent is preferably soluble CD4 or a derivative thereof specifically bindable with gp120. The stripping agents are preferably administered by bolus injection, more preferably by intravenous bolus injection.

The invention also provides an intravenous bolus injection of soluble CD4 or a derivative thereof specifically bindable with HIV gp120 in an amount sufficient to inhibit the virus for use in the treatment of HIV infection.

It has been found that sT4 strips gp120 from the HIV viral envelope and from the plasma membrane of infected cells, and that its inhibitory activity is competitive and irreversible. The ability of sT4 to bind and strip gp120 from the surface of HIV or HIV-infected cells significantly enhances the usefulness of sT4 as an anti-viral agent in AIDS therapeutics. If, as heretofore believed, sT4 activity in inhibiting virus infection of native T lymphocytes is due to coating of virus particles with sT4, it would act as a competitive, reversible inhibitor of viral interaction with target CD4 molecules. Effective therapy would be dependent upon mass action kinetics and require continuous exposure of the virus to saturating sT4 concentrations. The irreversible dissociation of gp120 from the viral envelope and plasma membrane of infected cells allows the stripping agent to be administered in bolus doses rather than by continuous infusion as is currently done. A bolus dose is a dose which is administered in a matter of seconds, e.g., less than about two minutes and usually in less than about ten seconds.

In preferred embodiments of the invention, the stripping agent is administered as a bolus injection, preferably intravenously. Administration of the stripping agent in this manner is convenient for patients. Administration can be accomplished in minutes, in contrast to the hours currently needed for administration of CD4 by continuous infusion. In preferred embodiments, administration of the stripping agent by intravenous bolus injection avoids the pain and bruising that often accompany intramuscular injection. This can be a significant advantage for persons who may have to receive the stripping agent daily, or at other frequent intervals, in addition to other treatments for HIV infection and related illnesses. Additionally, intravenous injection has the advantage that a greater percentage of the stripping agent will reach the target cells and virus than with intramuscular injection which relies on absorption into the bloodstream from surrounding tissues. This improved delivery efficiency is important because the administered dose of the stripping agent can be lowered without reduction in the efficacy of treatment.

Stripping agents useful in the methods of the invention are preferably soluble CD4 (or sT4) or fragments thereof having anti-HIV binding activity. Examples of stripping agents can be found in Smith et al ., Science 238 :1704 (1987); Deen et al ., Nature 331 :82 (1988); Traunecker et al ., Nature 331 :84 (1988); Fisher et al ., Nature 331 :76 (1988) and Hussey et al ., Nature 331 :78 (1988)

sT4, or fragments thereof, can be produced by standard recombinant DNA techniques A DNA coding sequence and amino acid sequence of sT4 are illustrated below.

```
                 10                    30                    50
     CAAGCCCAGAGCCCTGCCATTTCTGTGGGCTCAGGTCCCTACTGCTCAGCCCCTTCCTCC


                 70                    90                   110
     CTCGGCAAGGCCACAATGAACCGGGGAGTCCCTTTTAGGCACTTGCTTCTGGTGCTGCAA
                        MetAsnArgGlyValProPheArgHisLeuLeuLeuValLeuGln


                130                   150                   170
     CTGGCGCTCCTCCCAGCAGCCACTCAGGGAAAGAAAGTGGTGCtGGGCAAAAAAGGGGAT
     LeuAlaLeuLeuProAlaAlaThrGlnGlyLysLysValValLeuGlyLysLysGlyAsp
        -9                                -1 +1


                190                   210                   230
     ACAGTGGAACTGACCTGTACAGCTTCCCAGAAGAAGAGCATACAATTCCACTGGAAAAAC
     ThrValGluLeuThrCysThrAlaSerGlnLysLysSerIleGlnPheHisTrpLysAsn


                250                   270                   290
     TCCAACCAGATAAAGATTCTGGGAAATCAGGGCTCCTTCTTAACTAAAGGTCCATCCAAG
     SerAsnGlnIleLysIleLeuGlyAsnGlnGlySerPheLeuThrLysGlyProSerLys


                310                   330                   350
     CTGAATGATCGCGCTGACTCAAGAAGAAGCCTTTGGGACCAAGGAAACTTCCCCCTGATC
     LeuAsnAspArgAlaAspSerArgArgSerLeuTrpAspGlnGlyAsnPheProLeuIle


                370                   390          ′        410
     ATCAAGAATCTTAAGATAGAAGACTCAGATACTTACATCTGTGAAGTGGAGGACCAGAAG
     IleLysAsnLeuLysIleGluAspSerAspThrTyrIleCysGluValGluAspGlnLys


                430                   450                   470
     GAGGAGGTGCAATTGCTAGTGTTCGGATTGACTGCCAACTCTGACACCCACCTGCTTCAG
     GluGluValGlnLeuLeuValPheGlyLeuThrAlaAsnSerAspThrHisLeuLeuGln
                                                    104


                490                   510                   530
     GGGCAGAGCCTGACCCTGACCTTGGAGAGCCCCCCTGGTAGTAGCCCCTCAGTGCAATGT
     GlyGlnSerLeuThrLeuThrLeuGluSerProProGlySerSerProSerValGlnCys


                550                   570                   590
     AGGAGTCCAAGGGGTAAAAACATACAGGGGGGGAAGACCCTCTCCGTGTCTCAGCTGGAG
     ArgSerProArgGlyLysAsnIleGlnGlyGlyLysThrLeuSerValSerGlnLeuGlu
```

```
        610                630                     650
CTCCAGGATAGTGGCACCTGGACATGCACTGTCTTGCAGAACCAGAAGAAGGTGGAGTTC
LeuGlnAspSerGlyThrTrpThrCysThrValLeuGlnAsnGlnLysLysValGluPhe
151

        670                690                710
AAAaTAGACATCGTGGTGCTAGCTTTCCAGAAGGCCTCCAGCATAGTCTATAAGAAAGAG
LysIleAspIleValValLeuAlaPheGlyLysAlaSerSerIleValTyrLysLysGlu
                                  183

        730                750                770
GGGGAACAGGTGGAGTTCTCCTTCCCACTCGCCTTTACAGTTGAAAAGCTGACGGGCAGT
GlyGluGlnValGluPheSerPheProLeuAlaPheThrValGluLysLeuThrGlySer

        790                810                830
GGCGAGCTGTGGTGGCAGGCGGAGAGGGCTTCCTCCTCCAAGTCTTGGATCACCTTTGAC
GlyGluLeuTrpTrpGlnAlaGluArgAlaSerSerSerLysSerTrpIleThrPheAsp

        850                870                890
CTGAAGAACAAGGAAGTGTCTGTAAAACGGGTTACCCAGGACCCTAAGCTCCAGATGGGC
LeuLysAsnLysGluValSerValLysArgValThrGlnAspProLysLeuGlnMetGly

        910                930                950
AAGAAGCTCCCGCTCCACCTCACCCTGCCCCAGGCCTTGCCTCAGTATGCTGGCTCTGGA
LysLysLeuProLeuHisLeuThrLeuProGlnAlaLeuProGlnTyrAlaGlySerGly

        970                990                1010
AACCTCACCCTGGCCCTTGAAGCGAAAACAGGAAAGTTGCATCAGGAAGTGAaCCTGGTG
AsnLeuThrLeuAlaLeuGlyAlaLysThrGlyLysLeuHisGlnGluValAsnLeuVal

        1030               1050               1070
GTGATGAGAGCCACTCAGCTCCAGAAAAAATTTGACCTGTGAGGTGTGGGGACCCACCTCC
ValMetArgAlaThrGlnLeuGlnLysAsnLeuThrCysGluValTrpGlyProThrSer

        1090               1110               1130
CCTAAGCTGATGCTGAGCTTGAAACTGGAGAACAAGGAGGCAAAGGTCTCGAAGCGGGAG
ProLysLeuMetLeuSerLeuLysLeuGluAsnLysGluAlaLysValSerLysArgGlu

        1150               1170               1190
AAGGCGGTGTGGGTGCTGAACCCTGAGGCGGGGATGTGGCAGTGTCTGCTGAgTGACTCG
LysAlaValTrpValLeuAsnProGluAlaGlyMetTrpGlnCysLeuLeuSerAspSer

        1210               1230               1250
GGACAGGTCCTGCTGGAATCCAACATCAAGGTTCTGCCCACATGGTCCACCCCGGtgtaa
GlyGlnValLeuLeuGluSerAsnIleLysValLeuProThrTrpSerThrProValEnd
351                                                        369

        1270
tggcgcctctaga
```

Fragments of sT4 preferably comprise a gp120 binding domain from the V1 region of soluble CD4 (sCD4). Exemplary of such sT4 derivatives are polypeptides comprising the sT4 V1 domain or, preferably the V1 and V2 domains. Such sT4 derivatives, and methods for their production by recombinant DNA techniques are disclosed for example in co-pending EP-A-331,356, which is specifically incorporated as if fully set forth herein. sT4 or gp120-binding fragments thereof can also be administered as conjugates or fusions with other agents, such as IgG molecules or fragments thereof.

It is also within the scope of the invention to employ other types of compounds such as short peptides, antibodies or plant lectins that are capable of breaking the non-covalent bonds between gp120 and gp41 as stripping agents in the methods of the invention to strip gp120 from the virus or surface of infected cells. Preferred stripping agents, soluble T4 and fragments can be produced by purification from cells or body fluids or by recombinant DNA techniques.

Such stripping agents are useful in treating HIV infection in humans, i.e., in preventing or treating immune dysfunction associated with exposure or possible exposure to HIV infection. Prevention or treatment of immune dysfunction is readily monitored by clinical parameters, most notably T4 and T8 cell counts and ratios.

The stripping agents are administered in combination with a physiologically acceptable carrier or diluent such as saline solution or buffer at a physiologic pH. The stripping agents are administered preferably as an intravenous bolus injection. The frequency and dosage of the stripping agents will be dependent on such parameters as the stage of HIV infection, the binding affinity of the stripping agent for gp120, the age and weight of the infected individual and the presence of AIDS related diseases. It is expected that the stripping agent will be administered in doses ranging from about 1 mg to about 2 grams per day, more preferably from about 0.01 to about 1.0 gram per day. A typical administration protocol for initial treatment can be one or two daily intravenous injections of 1.0 to 100 mL of sT4 in 4.5% mannitol, 0.05% Tween 80 in 50 mM 1-histidine (pH 7) to administer about 0.001 to about 1.0 gram of sT4 per injection. The frequency and dosing can be adjusted thereafter depending upon the effects on T4 and T8 cell counts and ratios in patients as measured by standard clinical techniques. If T4/T8 cell counts and ratios do not improve, frequency and dosing can be increased by achieve a per day dosage of up to about 5 grams of sT4. Consecutive injections preferably are separated by at least about two hours and more preferably by at least 4 hours.

## Example 1

### Interaction of sT4 and HIV and HIV-infected cells.

To study the interaction of sT4 and HIV and HIV-infected CEM cells a conjugate of horseradish peroxidase (HRP) and sT4 was prepared after the method of Dickson et al ., Exp. Cell Res. 132 :488 (1981). The conjugate was purified by S-Sepharose affinity chromatography and S-200 HR size exclusion chromatography. The conjugate migrated as a single band of kd apparent mw 85.000 on SDS Page. Retention of gp120 binding activity was demonstrated using recombinant gp120 expressed in Drosophila cells. Despite retention of gp120 binding activity, the HRP-sT4 conjugate failed to give cytochemical staining of either HIV virus or of productively infected CEM cells. Similarly a colloidal gold conjugate of sT4 prepared after the method of DeMay, in Immunocytochemistry: Applications in Pathology and Biology J. Polck and S. Van Norden Eds. J. Wright and Sons, London, 1982, which also retained gp120 binding activity failed to bind to HIV or HIV-infected cells. The failure to demonstrate binding of HRP-sT4 or colloidal gold-sT4 with HIV or HIV infected cells suggested that sT4 may facilitate shedding of gp120 from HIV or HIV-infected cells.

### Surface density of glycoprotein spikes on the surface of mature HIV virus with and without prior treatment with sT4.

CEM cells sub-acutely infected with the HTLVIIIB strain of HIV (27 days post infection) were washed and incubated at 37° C for 1 hr in the presence of 10 $\mu$g/ml sT4 and then fixed in 2.5% glutaraldehyde in 0.1M phosphate buffer. Control cells were washed and fixed. Numerous mature HIV viruses remain associated with the cells despite repeated washing. Envelope glycoprotein spikes were revealed using the osmium-tannic acid-lead citrate method described by Ozel et al ., Arch., Virol, 100 :255 (1988). The linear surface density of envelope glycoprotein spikes (spikes/$\mu$m) was analyzed morphometrically from electron micrographs at 78,000 times magnification using a Zeiss Videoplan image anlaysis system. The linear surface density of envelope glycoprotein spikes was normally distributed in both control and sT4 treated viruses. However, treatment with sT4 resulted in a shift of the distribution. This change in distribution is reflected in a statistically significant decrease in the mean linear surface density of envelope glycoprotein spikes in the sT4 treated viruses (mean ± S.D. = 23 ± 11 spike/$\mu$m) compared to control mean ± S.D. = 28 ± 11 spikes/$\mu$m).

### Glycoprotein spike density in budding and mature virions.

To enhance the proportion of budding viruses, chronically infected CEM cells were co-cultured with uninfected CEM cells for five days. After five days, cells were rinsed in medium to remove free virus, incubated in medium alone or medium containing 10 $\mu$g/ml sT4 for 60 minutes at 37°C and fixed by the addition of an equal volume of 5% glutaraldehyde in 0.2 M phosphate buffer, pH 7.3. Cells were processed as described above and examined with an electron microscope (JEOL 100CX). In the control cells envelope projections (gp120) were clearly visible covering most of the surface of the budding virus and intermittently around mature virions. Following treatment with sT4, budding virions were seen to have fewer envelope projections in a non-uniform distribution on the surface of the virus. Mature particles were almost completely devoid of envelope projections.

The linear surface density of envelope glycoprotein spikes was significantly higher in budding virions compared to mature virions. Also, sT4 induced a greater degree of shedding of envelope glycoprotein spikes from budding virions (mean ± S.D. = 61 ± spikes/$\mu$m for sT4 treated; mean ± S.D. = 89 ± 23 spikes/$\mu$m for control, P <0.001, t test).

## Correlation of the loss of envelope glycoprotein spikes with loss of gp 120.

To remove free virus, $HTLV_{IIIB}$-infected CEM cells were washed with medium and incubated for 1 hr at 37°C in RPMI 1640 + 5% human AB serum in the presence or absence of 10-50 $\mu$g/ml sT4. Cell free supernatants were collected and treated with an equal volume of lysis buffer. Samples were electrophoresed through a 10% SDS-polyacrylamide gel and transferred to nitrocelulose. The samples were then treated with anti-gp120 antibodies or anti-HIV sera.

In a first series, nitrocellulose blots were reacted with a 1:1000 dilution of polyclonal rabbit anti-gp 120 and rabbit anti-CD4 followed by detection with a 1:3000 dilution of alkaline phosphatase conjugated goat anti-rabbit Ig antibodies. In a second series, nitrocellulose blots were reacted with a 1:500 dilution of anti-HIV sera followed by a 1:3000 dilution of alkaline phosphatase conjugated goat anti-human Ig antibodies.

Acutely infected cells treated with sT4 accumulated gp120 in the culture media, whereas, there was no detectable gp120 in the culture media of cells incubated in the absence of sT4. Additionally, when culture supernatants from sT4 treated cells were reacted with human anti-HIV sera there was no increase in viral gag proteins (p55, p24, p21, p17). These data indicate that sT4 strips gp120 from the surface of HIV and HIV-infected cells.

### Significance

Using electron microscopic cytochemistry, Applicants were unable to detect sT4 binding to either HIV or HIV-infected cells. Similarly following sT4 treatment of HIV and HIV-infected cells, Applicants have demonstrated a decrease in the surface density of HIV envelope glycoprotein spikes, as well as an increase in non-cell/virus associated gp120, without a concomitant increase in other viral proteins. Collectively, these data indicate that sT4 inactivates HIV by stripping gp120 from the surface of the virus.

### Example 2

### Bolus injection of sT4 in mice.

sT4 prepared in Chinese hamster ovary cells substantially as described by Maddon et al ., PCT WO 88/01304 was administered to CD-1 mice by intravenous bolus at 0.4, 4 and 40 mg/kg, 6 male mice/group. There were no adverse clinical signs and no effects on body weight or body weight gain. The mice were killed and autopsied 4 days after dosing. No drug-associated histopathologic changes were seen in liver, heart, lung, kidney, spleen, thymus or mesenteric lymph nodes. Serum samples collected at autopsy were assayed for the presence of anti-sT4 antibodies of the IgM class. A significant IgM response was found, indicating that sT4 is immunogenic in mice.

Male CD-1 virus antibody negative mice, supplied by Charles River Breeding Laboratories, Kingston, NY, were used in this study. The animals were approximately 4 weeks old when received. The animals were allowed to acclimate for 4 days prior to the start of the study. The animals were housed singly in stainless steel hanging baskets in a room maintained at 72 ± 4°F, 50 ± 10% humidity with an alternating 12-hour light/dark cycle and supplied with tap water from an automatic watering system and Certified Rodent Chow® #5002 (Lot Number Mar 17 8810, Purina Mills, Inc., St. Louis, MO) ad libitum .

A sT4 stock solution containing approximately 2 mg/ml of sT4 which was approximately 90% pure and 1.2-2.4 endotoxin units per ml was thawed by warming in the hand and aliquots were diluted 10- and 100-fold with Dulbecco's phosphate buffered saline (PBS) (pH 7) and were prepared immediately prior to dosing. Animals were dosed within 3 hours of thawing the stock solution. Controls received the PBS diluent.

Body weight data were recorded from all animals one day prior to treatment. On the day of treatment (day 1) animals were randomized by ranked body weight (22.0 - 24.8 grams), tattooed and assigned to one of four groups, with 6 animals per group. It was necessary to select three replacement animals from the remaining stock because of difficulties with intravenous dosing and the death of one control animal within 2 minute post-dosing. All animals received a standard injection volume of 20 ml/kg of PBS diluent (control), 100-fold dilute (low dose), 10-fold dilute (middle dose) or stock (high-dose) sT4. From the approximate concentration of the stock solution determined for an aliquot from this lot, these dilutions correspond to doses of 0.0, 0.4, 4.0 and 40.0 mg/kg. For dosing, animals were placed in a restrainer and their tails immersed in warm water to dilate their tail veins. On day 1, drug solutions or diluent were administered intravenously via lateral tail vein, over a period of less than 1 minute with a 1 ml syringe. After dosing, the animals were observed for clinical signs and body weights were recorded daily After recording their body weights on day 5, the animals were asphyxiated with $CO_2$ and decapitated. Blood samples for evaluating anti sT4 antibodies were collected from each animal and the following tissues collected and processed from all animals for histopathologic examination: heart, lungs kidney, liver (median lobe), mesenteric lymph node, thymus and spleen. Tissues from the high dose and control animals were examined by light microscopy. In addition, because a grossly apparent lesion was observed at autopsy, the left eye of one of the animals was collected, processed and examined by light microscopy.

Assay for Determination of Anti-sT4 Antibodies

Blood samples collected at autopsy were allowed to clot, the serum was separated by centrifugation and aliquots stored frozen at -70°C. The presence of anti-sT4 antibodies of the IgM class in these serum samples was assayed for with an enzyme linked immunosorbent assay (ELISA). Ninety-six well microelisa plates (Dynatech Immulon 1 Microelisa plates) were coated with 150 mg of sT4 by incubating overnight at 4C. Mouse IgM (5 µg/well, Calbiochem) was substituted for sT4 in some wells as a sensitivity control The wells were washed with Dulbecco's PBS and 0.5% Tween-20 (PBS-Tween) and then 100 µl of dilutions of serum from test mice (1:50 - 1:3200 in PBS-Tween) were added and incubated for 2 hours at 37°C. Diluent without added serum was substituted in some wells as a specificity control The plates were washed and 100 µl of a 1:1000 dilution of rabbit anti-rat IgG labeled with alkaline phosphatase (Zymed Labs, Inc.) was added to the wells and incubated for 1 hour at 37°C. Finally, the plates were washed and 100 µl of p-nitrophenyl phosphate,disodium (Sigma) 1 mg/ml in diethanolamine buffer were added. The plates were read at 410 nm at 15, 30, 45 and 60 minutes. Data were reported as the optical density at 410 nm ± standard error of the mean for 60 minutes in combinations, corrected for background, using the O.D. for the specificity control.

Body weight, change in body weight and anti-sT4 antibody titers were analyzed by the Least Squares Means and the Tukey's studentized range tests of the General Linear Models Procedure using SAS software, SAS User's Guide: Statistics. SAS Institute, Inc., Cary, North Carolina, 1982. A P value less than 0.05 was accepted as significant.

Clinical Signs

One PBS diluent control animal died within 2 minutes post-dosing, presumably due to cardiovascular stress from the relatively large injection volume of 20 ml/kg. This animal was replaced from stock. The replacement was given the same animal number. Other than transient blanching of the tail during injection, no other abnormal clinical signs were associated with dosing. Animals were observed for clinical signs through the 1.5 hours required for dosing and again 25 minutes after the last animal was dosed and no abnormal signs were observed. Animals were also observed once or twice daily on study days 2-4 and again no abnormal clinical signs were noted. On day 5, animals that had consistent body weight losses (control, one animal; low-dose, two animals; and middle dose, one animal had unkept hair coats. No other abnormal clinical signs were observed.

Body Weights

There were losses in body weight in two control, 3 low-dose, and one mid-dose animal between days 1 and 2. One control and two low-dose animals had losses in body weight that progressed through the period of the study. Despite these changes, there were no statistically significant differences between the groups on either day 2 or day 5. The observed individual animal differences are probably due to the stress associated with dosing with the relatively large volume of 20 ml/kg.

Anti-sT4 Antibodies

Forty mg/kg sT4 elicited a strong IgM response which was significant to a titer greater than 1:3200. Four and 0.4 mg/kg sT4 also elicited an IgM response. The magnitude of the response at these lower doses was the same and was lower than the magnitude of the response to 40 mg/kg at all dilutions; statistically significant levels of anti-sT4 IgM were detected to a dilution of 1:100.

Histopathology

There were no drug associated histopathologic changes found in the tissues examined.

The results of this study have shown that an intravenous bolus does of sT4 up to 40 mg/kg is well tolerated by mice. No overt effects were observed and there was no drug related effect on body weight nor on body weight gain. Likewise, no drug associated histopathologic changes were seen in the tissues examined. Because of the concentration of sT4 in the stock solution (2 mg/ml), 40 mg/kg was the maximum dose that could be administered at a dosing volume of 20 ml/kg. This is the maximum tolerable injection volume in 20 g mice.

The results also show that a single exposure to sT4 elicits an IgM antibody response which is dose-associated. As a recombinant human protein, sT4 was expected to be antigenic in mice as other recombinant human proteins intended for clinical use have been shown to be antigenic in rodents.

## Claims

1. A gp120 stripping agent for use in therapy.
2. A gp120 stripping agent for use in the treatment of HIV infection in humans.
3. The use according to claim 2 in which said stripping agent is soluble CD4 or a gp120 binding derivative thereof.
4. The use according to claim 2 or 3 in which the route of administration is intravenous.
5. The use according to claim 4 in which the route of administration is via an intravenous bolus.
6. The use according to claim 5 in which the stripping agent is sT4 and each bolus dose has 0.01 to 1.0 g of sT4.
7. The use of a gp120 stripping agent in the manufacture of a medicament for the treatment of HIV infection.
8. The use according to claim 7 in which the medicament is in the form of a bolus injection.
9. The use according to claim 8 in which the stripping agent is soluble CD4 or a gp120 binding derivative thereof.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X,D | NATURE vol. 337, 19 January 1989, LONDON pages 267 - 270; WATANABE,M.et al: "Effect of recombinant soluble CD4......" * the whole document * | 1-9 | A61K 37/02 |
| X,D | WO-A-8801304 (THE TRUSTEES OF COLUMBIA UNIVERSITY IN THE CITY OF NEW YORK) * page 68, line 15 - page 69, line 18; claims 23-33 * | 1-9 | |
| X,P | EP-A-330227 (THE TRUSTEES OF COLUMBIA IN THE CITY OF NEW YORK) * page 7, line 30 - page 8, line 15; claims 1-14 * | 1-9 | |
| X | WO-A-8901940 (BIOGEN INC) * page 9, lines 1 - 30 * * pages 24 - 27 * * pages 86 - 87 * | 1-9 | |
| X | WO-A-8903222 (DANA-FARBER CANCER INSTITUTE) * pages 48 - 53 * | 1-9 | TECHNICAL FIELDS SEARCHED (Int. Cl.5 ) |
| X | EP-A-176429 (INSTITUTE PASTEUR) * the whole document * | 1-9 | A61K |

The present search report has been drawn up for all claims

| Place of search | Date of completion of the search | Examiner |
|---|---|---|
| THE HAGUE | 27 AUGUST 1990 | FERNANDEZ Y BRA F. |